# EUROPEAN PATENT SPECIFICATION

(11) **EP 2 682 123 B1**
(45) Date of publication and mention of the grant of the patent: **03.07.2019**
(21) Application number: 12751975.9
(22) Date of filing: 10.04.2012
(51) Int. Cl.: A61K 36/73, A61K 36/28, A61K 36/03, A61P 5/00, A23L 33/105

(54) **BIOLOGICALLY ACTIVE FOOD ADDITIVE FOR NORMALIZING THE FUNCTION OF THE THYROID GLAND**
BIOLOGISCH WIRKSAMER NAHRUNGSMITTELZUSATZSTOFF ZUR NORMALISIERUNG DER FUNKTION DER SCHILDDRÜSE
ADDITIF BIOACTIF DESTINÉ À LA NORMALISATION DE LA FONCTION DE LA GLANDE THYROÏDE

(43) Date of publication of application: 08.01.2014
(73) Proprietor: Obshestvo S Ogranichennoj Otvetstvennostju "PARAFARM", Penza 440026 (RU)
(72) Inventor: TRIFONOV, Vyacheslav Nikolaevich, Penzenskaja obl. 442960 (RU); ELISTRATOVA, Julia Anatoljevna, Penza 440026 (RU); ELISTRATOV, Konstantin Gennadievich, Penza 440061 (RU); KURUS, Natalia Vyacheslavovna, Penza 440026 (RU)
(74) Representative: Engel, Christoph Klaus
(86) International application number: PCT/RU2012/000269
(87) International publication number: WO 2012/118412

(56) References cited:
- CN-A- 1 156 598
- RU-C1- 2 182 011
- RU-C1- 2 351 354
- US-A1- 2010 292 193
- DATABASE WPI Week 200927 Thomson Scientific, London, GB; AN 2009-H16520 XP002747961, & RU 2 351 354 C1 (MESHKOV P I) 10 April 2009 (2009-04-10)
- DATABASE WPI Week 200137 Thomson Scientific, London, GB; AN 2001-344345 XP002747962, & CN 1 156 598 A (CHENG X) 13 August 1997 (1997-08-13)
- DATABASE WPI Week 200877 Thomson Scientific, London, GB; AN 2008-N17278 XP002747963, & RU 2 333 764 C1 (MESHKOV P I) 20 September 2008 (2008-09-20)

## Description

This invention relates to a food processing industry, precisely to biologically active dietary additives (BAA) for the normalization of the thyroid function. The study of technical level on this matter demonstrated that it is known the patent Nº2333764 on an invention "Medication for the treatment and prevention of the disease of thyroid gland", containing roots and rhizomes of white cinquefoil (Potentilla alba L.), herb of bur beggar-ticks (Bidens tripartite L.) and roots of common licorice (Glycyrrhiza glabra L.)».

The patent No. 2351354 on an invention "Medication for the treatment and prevention of the disease of thyroid gland" is known. The medication is a gathered mix consisting of white cinquefoil, roots of common licorice, herb of bur beggar-ticks and thallome of devil's apron (Laminaria saccharina), with the following correlation of compounds - 20:5:8:7, wherein it contains roots and rhizomes of white cinquefoil or a top part of white cinquefoil, or their blend and might be performed in the form of reground vegetable powder, aqueous-alcoholic liqueur, liquid and dry extracts.

The objective of the invention is widening the range of biologically active dietary additives, possessing a wide spectrum of effect on the human body and contributing to the health improvement of the population.

This problem is solved by the suggestion of the biological active dietary additive for the normalization of the thyroid function that, according to the invention, contains roots and rhizomes of white cinquefoil or the top part of white cinquefoil, or their blend, as well as purple coneflower and devil's-apron (Laminaria), with the following correlation of the compounds (mass percent %):

| | |
|---|---|
| roots and rhizomes of white cinquefoil, top part of white cinquefoil, or their blend | 10-75, |
| purple coneflower | 10-50, |
| devil's-apron (Laminaria) | 10-80. |

The technical result of the invention is a reception of BAA with higher bioavailability, that does not have any side effects, with selected in an optimal way mixture ratio, that allows its usage in the most effective way for the prevention of diseases of the thyroid gland and the raise of body immunological status.

Thyroid disorders are accompanied by an immunity decrease; therefore, it was suggested to intensify the therapeutic effect of the white cinquefoil by the action of Echinacea purpurea. For that reason, Ltd. "Parapharm" offers to release biologically active additive "TIREO-VIT". "Tireo-Vit" contains white cinquefoil, that shows its antibacterial activity, contains a great number of macro- and microelements, normalizes the thyroid function, and it is used for the prevention of such thyroid disorders as thyrotoxicosis, hyperthyroidism, hyperplasia and hypotheriosis. There are two effective materials of the agent: elementary iodine and anion of iodous acid that is contained in a root of a white cinquefoil, and polysaccharides of Echinacea purpurea that have an immunopotentiating activity.

It is recommended to use as a biologically active dietary additive in the case of different thyroid disorders such as: hypothyroidism (hypofunction), hypethyroidism (hyperfunction, thyrotoxicosis, Basedow' disease, Graves' disease), autoimmune thyroditis (Hashimoto's thyroiditis, CAT), euthyroid goiter (diffusive, nodular/multinodular), hyperplasia of thyroid gland. Additional properties of medicinal plants included into "TIREO-VIT". White cinquefoil contains carbohydrates (amylum), iridoids, saponins, phenolcarbonic acids, flavonoids (quercetine) and tanning agents. The unique value of the plant is that it contains almost all Mendeleev's periodic table. First of all a mineral balance and salt exchange within the body tern to normal while using a White cinquefoil, a hormonal balance straightens; metabolic processes normalize. All agents contained in cinquefoil are not toxic and do not have any side effects on the body.

Flavonoids (P-vitamin remedies) have a positive impact on the blood vessel walls, regulating their ductance, eliminating their brittleness and fragility; they reduce heart rate and increase their amplitude; reduce arterial tension, influence on the blood count, strengthen the production of erythrocytes and leukocytes, reducing the level of cholesterol; they intensify a bile flow and decrease the tone of intestinal canal. Phenolcarbonic acids (pyrocatechins, hydroquinones and pyrogallols) have a broad capacity for an inversive oxidation with the formation of semiquinone radicals and quinones, thereby they easily react with thiol groups of protein; particularly, their influence on vascular and cellular permeability might be explained by the cooperation of these agents with proteins of plasma membranes and vassal-tissue barriers.

Iridoids-phytochemicals provide a therapeutic effect in case of thyroid gland disorders. It has been proved, that iridoids kill very harmful free radicals, maintain normal level of cholesterol, increase energy, stimulate cardiac performance, reinforce immune system, prevent the body of various kinds of inflammations, keep the cells away from mutation and increase healthy brain activity.

White cinquefoil is the concentrate of Mn, Zn, Cu, Se, Co, Fe, Si, Al. White cinquefoil demonstrates its antibacterial activity, and for that reason it is applied in case of colitis, enterocolitis, dysentery, diarrhea, gastrointestinal torminas as anastaltic and hemostatic remedy. Besides that, herbalists recommend to use white cinquefoil as a preventive measure and treatment of hepatic disorders, cardiovascular system and gastrointestinal tract, particularly, ulcer, as well as antiseptic and wound-healing remedy. White cinquefoil is used in the case of arthragra, rheumatism, it is a great blood depurant. White cinquefoil is a reliable remedy in the case of ovarian dysfunction, descent of womb and dysmenorrhea. The intake of white cinquefoil regulates the arterial blood pressure; normalizes a body weight; regulates calcium exchange in the body, accordingly it reverses the condition of skin, hair and nails. It contributes to the resolution of soft tumors, palpable abnormalities; it has been proved that white cinquefoil removes radiation, reduces the level of cholesterol, resolves cystics and myomas.

Echinacea purpurea contains essential oils, glycosides, amides, antibiotic polyacetylenes, inulin, vitamins B group, iron, that is needed to form red corpuscles, calcium, needed for strong bones and teeth, selenium for the capacity to resist diseases, as well as silicium that is needed to form tissues. Echinacea is used in the case of disorders associated with a reduction of the functional status of immune system, that were induced by chronic inflammatory diseases, influence of ionizing radiation, ultraviolet rays, chemotherapeutic compounds and long-term antibiotic therapy. At the intake of Echinacea containing medications related to some metabolic disorders (diabetes and hepatic disorders), influence of various chemical compounds of toxic nature, contained in the air and food substances (heavy metals, pesticides, insecticides and fungicides), the stimulation of immune system occurs. As well Echinacea's medications have antibacterial, anti-viral and anti-mycotic properties. Echinacea's medications inhibits the growth and reproduction of streptococcus, staphylococcus, Escherichia coli, viruses of influenza, herpes, stomatitis; they are effective in the case of inflammatory diseases (rheumatism, polyarthritis, prostatitis and gynecological disorders), upper air passages diseases; in the case of a various wound processes (trophic ulcers and central osteitis) and microbial eczema. This declared BAA is produced in a powdered, tableted or capsular form, as well as in the form of aqueous alcoholic extract and forms made on the basis of this extract, more specifically: powder, tablets or capsules.

Devil's apron (Laminaria) (sea girdle) - iodine (around 3%) in a form of iodine-organic compounds and iodides; vitamins A, B₁, B₂, B₁₂, C, and O, folic acid, carbohydrates (polysaccharide aminarine, marmitol, alginic acid), aging pigment, bromine salts, traces of arsenic, salts of potassium, natrium, calcium, magnesium, cuprum and cobalt. Due to the great content of iodine and vitamins it is used in a form of powder for the treatment and prophylaxis of atherosclerosis and Derbyshire neck. In oncological clinic it has been diagnosed that the powder of laminaria contributes to the improvement of a general state of the patients and frame of their mind (A. D. Turova, A. S. Gladkikh, 1965).

It is recommended in case of chronic constipations as eccoprotic (2-3 g of powder for 150 ml of water, for a night), as well as for the improvement of metabolic process and in the case of arthraga. In China and Japan laminaria is used in treatment and prophylaxis of the thyroid disorders. Combined administration of white cinquefoil and Echinacea is highly reasonable. Common usage of the specified plants in treatment of thyroid function disorders in the prior art is not known.

Both plants increase energy and they are immune response modifiers, activators of cell-mediated immunity and intrinsic cellular metabolism. Common use of these two plants will substantially intensify recited pharmacological effects. Thus, a maximum activity of Echinacea appears strictly subjected to availability of bioflavonoids which are contained in white cinquefoil.

Incorporation of laminaria into the agent's content is subjected to the fact that laminaria is rich with organic iodine which is needed to achieve a maximum of therapeutic effect, especially in case of hypothyroidism and euthyroid goiter. As a result of numerous experiments we have found that to receive an additive with the supreme bioavailability of all included compounds, as well as for the purpose of reception of the maximum efficacy of this additive in respect of prevention of thyroid gland disorders and raising the immunological status, the balance of the compounds has to be the following: white cinquefoil (roots and rhizomes or the top part, or their blend) - 10-75 in mass percent (%), Echinacea purpurea 10-50 in mass percent (%), laminaria 10-80 in mass percent (%). Invention has been illustrated with the following examples:

### Example 1:

To prepare BAA the compounds are combined in the ratio: 50 mg of reground roots of white cinquefoil, 10 mg milled herb of Echinacea purpurea and 10 mg of milled thallomes of Laminaria. All ingredients are thoroughly mixed. The received blend is pelletized.

### Example 2:

To prepare BAA the compounds are combined in the ratio: Leaves of white cinquefoil - 50 mg, herb of Echinacea purpurea - 50 mg and thallomes of Laminaria - 100 mg. All ingredients are milled and accurately mixed. The received blend is encapsulated. BAA constructed in accordance with the 1^{st} example has participated in the approbation on 10 volunteers: pregnant women who in the consequence of examination were for the first time diagnosed with hypothyroidism, nodosties of thyroid gland and reduced immunity. For the period of pregnancy the women have been taking BAA on the model of 1^{st} example 3 times per day. The pregnancy of all women was without any complications, and the labours were without complications as well. Results of the analyses after 6 months of the intake of BAA on the model of 1^{st} example demonstrated that this BAA has led the thyroid gland to euthyroid state and substantially improved immunity. The usage of BAA did not exert any side effects on pregnant women.

The same results have been received after the approbation of BAA to the food, where the quantitative ratio of the compounds was chosen out of claimed limits. Thus, the offered BAA owing to an effectively selected qualitative and quantitative ratio of the compounds allows providing an effective prophylaxis of thyroid gland disorders and raising the immunological status without any side effects.

## Claims

1. A biologically active food additive (BAA) for normalizing the function of the thyroid gland containing the roots and rhizomes of white cinquefoil (Potentilla alba) or a top part of white cinquefoil, or their blend as well as Echinacea purpurea and Laminaria at the following compounds correlation, in mass percent (%):
| | |
|---|---|
| roots and rhizomes of white cinquefoil, or top part of white cinquefoil, or their blend | 10-75, |
| Echinacea purpurea | 10-50, |
| devil's-apron (Laminaria) | 10-80 |

## Patentansprüche

1. Biologisch aktiver Nahrungsmittelzusatzstoff (BAA) zur Normalisierung der Schilddrüsenfunktion, welcher die Wurzeln und Rhizome von weißem Fingerkraut (Potentilla alba) oder einem oberen Teil von weißem Fingerkraut enthält, oder einer Mischung aus diesen; sowie Purpur-Sonnenhut und Zuckertang (Laminaria) in folgendem Mischungsverhältnis in Masse-%:
| | |
|---|---|
| Wurzeln und Rhizome aus weißem Fingerkraut, oder oberem Teil eines weißen Fingerkrauts, oder einer Mischung aus diesen | 10-75, |
| Purpur-Sonnenhut | 10-50, |
| Zuckertang (Laminaria) | 10-80 |

## Revendications

1. Additif alimentaire actif biologiquement (BAA) destiné à normaliser le fonctionnement de la glande thyroïde contenant les racines et rhizomes de potentille blanche (Potentilla alba) ou une partie supérieure de potentille blanche, ou leur mélange ainsi que de l'Echinacea purpurea et de la Laminaria à la corrélation de composé suivante, en pourcentage massique (%) :
| | |
|---|---|
| racines et rhizomes de potentille blanche, ou partie supérieure de potentille blanche, ou leur mélange | 10-75, |
| Echinacea purpurea | 10-50, |
| laminaire sucrée (Laminaria) | 10-80. |
